# EUROPEAN PATENT SPECIFICATION

(11) **EP 0 523 911 B1**
(45) Date of publication and mention of the grant of the patent: **20.09.1995**
(21) Application number: 92306295.4
(22) Date of filing: 09.07.1992
(51) Int. Cl.: A61K 7/48, A61K 7/50

(54) **Three phase silicone containing cosmetic composition**
Drei-Phasen kosmetisches Präparat mit Silikonen
Composition cosmétique à trois phases contenant des silicones

(30) Priority: 18.07.1991 GB 9115591
(43) Date of publication of application: 20.01.1993
(73) Proprietor: DOW CORNING GmbH, D-65091 Wiesbaden (DE)
(72) Inventor: Roidl, Josef Jakob, W-6501 Saulheim (DE)
(74) Representative: Vandamme, Luc Johan Roger

(56) References cited:
- GB-A- 1 158 155

## Description

This invention is directed to a cosmetic composition in the form of a multi-phase system which upon standing separates into three distinct layers. More particularly, silicone fluids constitute the upper and lower layers and the silicone layers are separated by an aqueous phase. The silicone oil layers remain distinct from one another in that the lower layer is insoluble in the upper layer.

The use of three phase systems in cosmetic applications is not new. Likewise, the use of silicone fluids in such three phase cosmetics is not new. For example, in German patent specification 30 09 763 is described a cosmetic composition consisting of 3 liquid phases, the liquids of which are immiscible with one another. A first phase consists of water and dissolved materials which are insoluble or only sparingly soluble in alcohol. A second phase has a water repellant solvent, e.g. a silicone oil, with optionally dissolved materials therein. A third phase is immiscible with both other phases, e.g. certain alcohols. In G.B. Patent Application 2 206 048 is described a three layer cosmetic which contains (i) an aqueous phase, (ii) a volatile silicone phase and (iii) a phase which is a silicone emulsifying agent. In European Patent Specification 422,984 is described a 3-phase system consisting of a perfluorated etherphase, an aqueous phase and a silicone phase comprising at least a siloxane polyoxyalkylene copolymer and a methyl substitued cyclic siloxane.

The present invention while containing an aqueous phase and a volatile silicone phase, differs from G.B. 2 206 048 and from EP 422,984 in that no emulsifying agent is employed. This provides the advantage that any tendency due to skin irritation from an emulsifier is eliminated. Instead of a silicone emulsifying phase as in the cited patent applications, this invention utilizes a fluorosilicone fluid which is substantive to the skin. The advantage of the fluorosilicone is that it functions as a moisturiser and forms a film on the skin which acts as a barrier against transepidermal water loss, with the result that the skin tends to be maintained in a softened condition. The present invention also provides the advantage that a varied degree of freedom exists for the formulation of products. Thus, each layer functions as a carrier for hydrophobic and hydrophilic adjuvants which may be included in each of the layers depending upon compatibility with a particular layer. End products in accordance with the present invention can be formulated as bath oils for addition to bath water or they can be used in the form of a lotion for applying to the skin.

These and other features, objects and advantages of the present invention will become more apparent upon a consideration of the following detailed description.

In accordance with the present invention, there is provided a composition which has at least three separate and distinct phases. The first phase is a low viscosity volatile organopolysiloxane, the second phase is an aqueous phase and the third phase is a fluorosilicone fluid. The composition upon reaching equilibrium by remaining in an undisturbed state forms three layers. The low viscosity volatile organopolysiloxane will occupy the uppermost layer, the aqueous phase will occupy the middle layer and the fluorosilicone fluid will occupy the lowermost layer.

The fluorosilicone fluid has the average formula R₃SiO(RR′SiO)ₓSiR₃ or R₃SiO(RR′SiO)ₓ(R₂SiO)_{y}SiR₃ wherein R is a monovalent hydrocarbon radical, R′ is a fluoroalkyl radical, x is an integer having a value of 5 to 100 and y is an integer having a value of zero to about fifty. The monovalent hydrocarbon radical R can be an alkyl radical such as methyl, ethyl, butyl, propyl and the like; an alkenyl or cycloalkenyl radical such as vinyl, allyl, cyclopentenyl and the like; an aryl radical such as phenyl, tolyl, xylyl and the like; an arylalkyl radical such as beta-phenylethyl, beta-phenylpropyl and the like; or a cycloaliphatic radical such as cyclohexyl, cyclopentyl, cycloheptyl and the like. Most preferably R is a methyl radical. The fluoroalkyl radical R′ in the above formula has the general formula (CₘF₂ₘ₊₁)(CH₂CH₂)_{z} wherein m is an integer having a value of 1 to 4 and z is an integer having a value of 1 to 3. Suitable fluoroalkyl radicals include CF₃CH₂CH₂-, CF₃CF₂CH₂CH₂- and CF₃CF(CF₃)CH₂CH₂-. The preferred fluoroalkyl radical is CF₃CH₂CH₂-. Such fluorosilicone fluids can be made by methods described in U.S. Patent No. 2,961,425.

While the preferred fluorosilicone fluid may be any polyfluoroalkylmethylsiloxane, most preferred is polymethyl-3,3,3-trifluoropropylsiloxane having the formula: 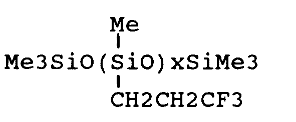
in which Me is methyl and x is an integer selected to provide a fluid having an average molecular weight of 500 to 15000. These materials are commercially available in viscosities ranging from 300 to 10,000mm²/s measured at 25°C. Preferred in accordance with the present invention is a fluorosilicone fluid having a viscosity of 300mm²/s.

The volatile low viscosity organosilicon compound contemplated in accordance with the present invention include both cyclic silicone fluids and linear silicones. Representative of these volatile silicone materials are polydimethylcyclosiloxane and hexamethyldisiloxane. Such fluids have viscosities ranging from 0.65 to 5.0mm²/s measured at 25°C.

The volatile cyclic silicones conform to the formula (R˝₂SiO)ₓ in which R˝ is an alkyl radical having from one to three carbon atoms or a phenyl group. Most typically the cyclic siloxanes have the formula [(CH₃)₂SiO]ₓ in which x is an integer from three to ten. Some volatile cyclic siloxane compounds found to be especially useful in accordance with the present invention are the tetramer compound octamethylcyclotetrasiloxane and the pentamer compound decamethylcyclopentasiloxane. Mixtures of the tetramer and pentamer may also be employed. Such cyclic siloxanes have viscosities ranging from about 2.5mm²/s to about 5mm²/s. These materials are described under The Cosmetics, Toiletries and Fragrance Association designation as cyclomethicone.

The volatile low viscosity linear silicone fluids have the formula R²₃SiO(R²₂SiO)ₙSiR²₃ in which R² is an alkyl radical having one to six carbon atoms and n is an integer of from two to nine. Most representative of this class of linear siloxane is hexamethyldisiloxane of the formula 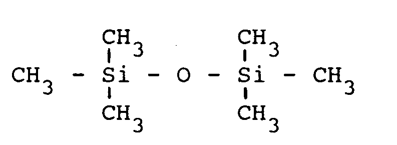
which has a viscosity of 0.65mm²/s measured at 25°C.

Both the cyclic and linear low viscosity volatile silicones are optically clear fluids and are essentially odorless, nontoxic, nongreasy and nonstinging. Cosmetically they are nonirritating to the skin and possess the properties of good spreadability and ease of rub-out. The materials evaporate leaving behind no residue. These materials are commercially available and methods for their preparation are well known in the art.

In addition to the three materials which constitute the separate layers each layer may contain other adjuvants which provide a more cosmetically acceptable product. Thus, the aqueous phase may be modified to include hydrophilic materials, while the volatile silicone phase and the fluorosilicone phase may be modified to include materials which are hydrophobic. Further, the volatile silicone phase especially may contain other organosilicon compounds in particular phenylfunctional polysiloxanes. A particularly suitable example thereof are polyphenylmethylsiloxanes which are available in viscosities of e.g. 22mm²/s. They provide the advantages and benefits of establishing on the skin durable water repellency and skin protection especially against water-borne irritants. Such phenylfunctional polysiloxanes additionally provide a non-oily easy-to-spread emolliency to the skin and allow natural skin transpiration through a soft invisible film. Added skin lubricity and softness are provided by these polyphenylmethylsiloxanes.

The upper volatile silicone layer is capable of functioning as a carrier for hydrophobic oily components such as natural vegetable oils; animal derived lipids such as lanolin and mink oil; low melting fatty alcohols such as oleyl and hexadecyl alcohol; low melting synthetic glycerides such as glyceryl monooleate and glycerol monolaurate and mineral oils. The upper layer is also adapted to contain an oil compatible dye or colour, perfumes, sunscreens, fats and waxes. The upper layer including the adjuvant materials constitutes about 1 to 50 percent by weight of the composition.

The aqueous phase acts as a separation between the two silicone phases. In addition to water it may contain water soluble materials such as water soluble dyes and colours, vitamins, acids for control of the pH, glycerine and glycerine derivatives, propylene glycol, sorbitol, preservatives, humectants, moisturizers and sunscreens. Vitamins include A, D, E, K, B1, B2, B12, B15, B17, C, niacin, folic acid, panthotenic acid, biotin, bioflavinoids, choline, inositol and F. Cod liver oil and retinoids may also be included. Among the various preservatives which may be employed are p-hydroxybenzoates, dehydroacetic acid, sorbic acid, imidozolidinylurea, 2-bromo-2-nitro-1,3-propane diol, formaldehyde and 1-(3-chloroallyl)-3,5,7-triaza-1-azoniaadamantane chloride. Suitable dyes and colours are natural dyes such as alkanet, annatto, carotene, chlorophyll, cochineal, saffron, and henna. Inorganic colorants include ochre, umber, sienna, iron oxides, chrome oxide greens, and ultramarine pigment blues and pinks. Dyes and colorants carried under the nomenclature designation "D&C" are also appropriate such as D&C red No. 2 and D&C orange No. 4. The aqueous layer including the adjuvant materials constitutes about 1 to 40 percent by weight of the composition.

The lowermost fluorosilicone layer is substantive to the skin and may include a dye or colour. A particularly preferred colorant is the commercial product GRAS W 7200 which provides a blue hue. This colorant is available as a 0.2 percent solution in paraffin oil and can be used in the fluorosilicone phase in an amount of about two percent. The fluorosilicone layer is capable of forming a film on the skin which acts as a barrier against the passage of solvent materials and organic oils. The fluorosilicone layer is not soluble in the uppermost volatile silicone layer and vice versa, and hence separation is possible between these two organosilicon phases. The lowermost fluorosilicone layer including the adjuvant materials constitutes about 1 to 60 percent by weight of the composition. Substantivity and durability of the fluorosilicone compound was verified by using a two gram sample of polymethyl-3,3,3-trifluoropropylsiloxane stirred into a bath of twenty litres of warm water. The forearm of a volunteer was placed in the bath for 30 seconds. The forearm was rinsed with running water and dried with paper towels without rubbing. After two minutes the presence of the fluorosilicone on the forearm was shown by Fourier Transform Infrared Spectrophotometric analysis (FTIR).

The cosmetic composition of the present invention may be applied to the body as a lotion or it may be used as a bath oil. The floating layer of perfumed oil affords a pleasant fragrance and a desirable feel to the skin. The oleaginous film adheres to the body surfaces and imparts a hydrophobic barrier to the skin keratin.

Examples of emollients and moisturisers which may be used in this invention include straight, branched or cyclic hydroxy compounds such as alcohols containing 1 to 30 carbon atoms; straight, branched or cyclic carboxylic acids containing 1 to 31 carbon atoms; acid esters containing C₁-C₃₀ carboxylic acids esterified with C₁-C₃₀ alcohols; alcohol ethers containing 1 to 30 carbon atoms; alkanes of the formula H-(CH₂)ₙ-H wherein n is 5 to 30 and siloxanes. Examples of such functional materials include 2-ethylhexyl oxystearate; arachidyl propionate; 2-ethylhexyl adipate; isopropyl myristate; ethanol; stearyl alcohol; propylene glycol; proprionic acid; stearic acid; polyoxypropylene cetyl alcohol; polyoxypropylene lanolin alcohol; Carbowaxe® 300; petroleum jelly; mineral oil; aliphatic hydrocarbons such as mineral spirits; lanolin and lanolin derivatives such as acetylated lanolin and isopropyl lanolate; glycerine and glycerine derivatives; linear polydi-methylsiloxane; polyphenylsiloxane and polydimethyltrimethylsiloxane.

The compositions of the present invention may include any type of fragrance, cologne or perfume compatible with the materials. For example the fragrance may be a natural product such as Ambergris, Benzoin, Civet, Clove Leaf Oil, Galbanum, Jasmine Absolute, Labdanum, Mate′, Melilot, Mimosa, Musk Tonquin, Myrrh, Mousse de Chene, Olibanum, Opopanax, Orris, Patchouli, Rosemary Oil, Sandalwood Oil, Vertivert Oil and Violet Leaves Absolute. Among the various aroma chemicals that may be employed in addition to the foregoing natural products are, for example, acetylated cedarwood terpenes, amylcinnamic aldehyde, amyl salicylate, methyl salicylate, benzyl acetate, benzyl salicylate, p-tert-butylcyclohexyl acetate, citronelol, coumarin, Galaxolide, geraniol, hexylcinnamic aldehyde, isobornyl acetate, linalool, linalyl acetate, Lyral, musk ambrette, phenethyl alcohol, tetrahydromuguol and terpinyl acetate. Fragrances that have become classics as descriptors for other fragrances in the same family are also included herein and would comprehend the Straight Floral Family, Floral Bouquet Family, Aldehydic Floral Family, Oriental Family, Chypre Family, Woody Family, Green Family, Citrus Family, Fougere Family, Canoe Family, Musk Family, Animal Family, Leather Family, Spice Family and the Herbal Family.

Preferred fragrances include Citronellol, Cineole, YSL PARIS®, manufactured by Charles of the Ritz, Group of New York, New York, U.S.A.; JOY® manufactured by Jean Patou Inc. of New York, New York, U.S.A.; OSCAR de la RENTA® manufactured by Oscar de la Renta Limited of New York, New York, U.S.A. and IVOIRE de BALMAIN™, manufactured by Balmain International B.V. of Rotterdam, Netherlands.

It is intended that any sunscreen active compound be comprehended within the scope of the present invention. Sunscreens are evaluated according to their ability to slow the erythema or sunburn resulting from the exposure of skin to ultraviolet light between about 290 to 320 nanometers (the UV-B region). This is accomplished by absorbing damaging radiation before the radiation contacts the skin surface. Paraaminobenzoic acid derivatives and cinnamates such as octyl methoxycinnamate are two of the currently most preferably and commercially employed categories of sunscreen active compounds. UV-A region agents capable of absorbing ultraviolet light in the range of 320 to 400 nanometers are also useful in accordance with the present invention, including benzophenones and materials such as butyl methoxy dibenzoylmethane. Some additional examples of sunscreen chemicals which may be employed in accordance with the present invention are 2-ethoxyethyl p-methoxycinnamate; methyl anthranilate; homomenthyl salicylate; glyceryl p-aminobenzoate; isobutyl p-aminobenzoate; isoamyl p-dimethylaminobenzoate; 2-hydroxy-4-methoxybenzophenone-5- sulphonic acid; 2,2′-dihydroxy-4-methoxybenzophenone; 2-hydroxy-4-methoxybenzophenone; 4-mono and 4-bis(3-hydroxypropyl)amino isomers of ethyl benzoate and 2-ethylhexyl p-dimethylaminobenzoate.

Although not required it is preferred to employ an acid to adjust the pH within the range of 5 to 9, or more preferably within the range of 6 to 8, in the compositions of this invention. Any water soluble acid such as carboxylic acid or a mineral acid is suitable. For example, suitable acids include mineral acids such as hydrochloric, sulphuric and phosphoric; monocarboxylic acid such as acetic acid, lactic acid or propionic acid and polycarboxylic acids such as succinic acid, adipic acid and citric acid.

The cosmetic composition of the invention is applied by firstly dispensing the phases in each other. This can easily be achieved by simply shaking the container in which the composition is provided. The dispersion of the 3 phases is sufficiently stable to allow application of the composition in the normal way, e.g. by pouring, dabbing, sprinkling or rubbing.

The following examples illustrate the concept of the present invention.

### Example I

Into three separate vessels were placed equal volumes of water, a fluorosilicone fluid and a volatile cyclic siloxane. The fluorosilicone fluid was polymethyl-3,3,3-trifluoropropylsiloxane which had a viscosity of 300mm²/s. The cyclic siloxane was a mixture of octamethylcyclotetrasiloxane and decamethylcyclopentasiloxane. To each of the vessels containing an organosilicon compound there was added two percent by weight of a blue pigment known as "Vert au Gras W 7200". The blue pigment was in the form of a 0.2 percent by weight basic solution in paraffin oil. This pigment is a product of Wackherr (les Colorants), 55 Voie des Bans, 95100 Argenteuil, Val-d'oise, France. The contents of the two organosilicon compound containing vessels were stirred in order to disperse the pigment in the vessel contents. The contents of each of the three vessels was poured into a glass bottle and the glass bottle was allowed to remain undisturbed. A three phase composition formed in the glass bottle. The blue-hued volatile siloxane occupied the upper phase, the water occupied the middle phase and the blue-hued fluorosilicone fluid occupied the lower phase. The three phase composition was found to be useful as a bathing water additive and provides protection and softness to the skin. While any volume ratio of the phases can be employed in accordance with the present invention the preferred volume ratio of the three phases as shown in this example is 1:1:1.

### Example II

Example I was repeated with the same results except that the pigment used was two percent by weight of the blue pigment "Vert au Gras W 7200" and two percent by weight of the pigment "Jaune au Gras W 1202", both of which were in the form of a 0.2 percent by weight basic solution in paraffin oil. Both pigments are products of Wackherr (les Colorants), 55 Voie de Bans, 95100 Argenteuil, Val-d'oise, France.

### Example III

The substantivity on skin of the fluorosilicone fluid used in Examples I and II was confirmed by use of Fourier transform infrared spectroscopy with attenuated total reflectance (FTIR) in accordance with the procedure described in the Journal of the Society of Cosmetic Chemists, Volume 37, Pages 73 to 87, March/April 1986. In this procedure the test site selected was the volar forearm of a volunteer on which a rectangular area of about 80cm² was marked. A sample test solution of the fluorosilicone compound was prepared and constitutued a ten weight percent solution of the fluorosilicone compound in cyclomethicone. A soap mixture was prepared containing 0.5 weight percent of IVORY bar soap in distilled water. A wash/rinse procedure of 15 soap rubs and 10 water rinses per cycle was employed. The test began by soap washing and rinsing the test site. The test site was blotted dry and after one minute the test site was softened by applying to the test site a water soaked towel for one minute. The test site was placed on an ATR prism and a back-ground scan was collected for the test site. A known weighed quantity of the test solution was applied to the test site with a small brush. The quantity of the test solution which was applied to the test site was determined by calculating the weight difference between the vial of solution plus the brush before and after application of the test solution to the skin. A maximum loading of 10 to 12 milligrams of the fluorosilicone fluid on the 80cm² area was employed. The solvent was allowed to evaporate for 15 to 30 minutes. The test site was softened and an IR scan was collected in order to determine an initial condition. The test site was soap washed, rinsed, blotted dry and softened as described above and IR scans were collected for three of the wash cycles. Quantitation of fluorosilicone fluid on the skin was calculated. The results of the calculations confirmed that the fluorosilicone fluid was highly substantive to the skin.

## Claims

1. A cosmetic composition comprising at least three separate and distinct phases, characterised in that the first phase comprises a low viscosity volatile organopolysiloxane, the second phase is an aqueous phase and the third phase comprises a fluorosilicone fluid.

2. A composition according to Claim 1 in which the fluoro-silicone fluid is a polyfluoroalkylmethylsiloxane.

3. A composition according to Claim 1 or 2 in which the fluoro-silicone fluid is polymethyl-3,3,3-trifluoropropyl-siloxane having a viscosity of between 300 and 10,000 mm²/s at 25°C.

4. A composition according to any one of the preceding claims in which the low viscosity volatile organopolysiloxane comprises a cyclic organopolysiloxane having the formula [(CH₃)₂SiO]ₓ in which x is an integer having a value of from three to ten.

5. A composition according to Claim 4 in which the low viscosity volatile organopolysiloxane is a mixture of octamethylcyclotetrasiloxane and decamethylcyclopentasiloxane.

6. A composition according to any one of the preceding claims in which the low viscosity volatile organopolysiloxane comprises a linear organopolysiloxane having the formula R²₃SiO(R²₂SiO)ₙSiR²₃ in which R² is an alkyl radical having from one to six carbon atoms and n is an integer having a value of from two to nine.

7. A composition according to any of the preceding claims in which the first phase includes a phenylfunctional polysiloxane.

8. A composition according to any one of the preceding claims in which the first and/or the third phases comprise also hydrophobic materials and in which the second phase also comprises hydrophilic materials.

9. A composition according to any one of the preceding claims in which the first, second and third phase constitute respectively 1 to 50%, 1 to 40% and 1 to 60% by weight of the total weight of the composition.

10. A method of cosmetically treating human or animal bodies comprising applying to the bodies a composition according to any one of the preceding claims.

## Patentansprüche

1. Kosmetikzusammensetzung, umfassend mindestens drei getrennte und verschiedene Phasen, dadurch gekennzeichnet, daß die erste Phase ein flüchtiges Organopolysiloxan niedriger Viskosität umfaßt, die zweite Phase eine wäßrige Phase ist und die dritte Phase ein Fluorsilikonöl umfaßt.

2. Zusammensetzung nach Anspruch 1, worin das Fluorsilikonöl ein Polyfluoralkylmethylsiloxan ist.

3. Zusammensetzung nach Anspruch 1 oder 2, worin das Fluorsilikonöl Polymethyl-3,3,3-trifluorpropylsiloxan mit einer Viskosität von 300 bis 10.000 mm²/s bei 25°C ist.

4. Zusammensetzung nach einem der vorhergehenden Ansprüche, worin das flüchtige Organopolysiloxan mit niedriger Viskosität ein cyclisches Organopolysiloxan der Formel [(CH₃)₂SiO]ₓ umfaßt, worin x eine ganze Zahl mit einem Wert von 3 bis 10 ist.

5. Zusammensetzung nach Anspruch 4, worin das flüchtige Organopolysiloxan mit geringer Viskosität eine Mischung aus Octamethylcyclotetrasiloxan und Decamethylcyclopentasiloxan ist.

6. Zusammensetzung nach einem der vorhergehenden Ansprüche, worin das flüchtige Organopolysiloxan geringer Viskosität ein lineares Organopolysiloxan der Formel R²₃SiO(R²₂SiO)ₙSiR²₃ umfaßt, worin R² ein Alkylrest mit 1 bis 6 Kohlenstoffatomen ist und n eine ganze Zahl mit einem Wert von 2 bis 9 ist.

7. Zusammensetzung nach einem der vorhergehenden Ansprüche, worin die erste Phase ein Polysiloxan mit Phenylfunktionen einschließt.

8. Zusammensetzung nach einem der vorhergehenden Ansprüche, worin die erste und/oder dritte Phase auch hydrophobe Materialien enthält und worin die zweite Phase auch hydrophile Materialien enthält.

9. Zusammensetzung nach einem der vorhergehenden Ansprüche, worin die erste, zweite und dritte Phase 1 bis 50 Gew.-%, 1 bis 40 Gew.-% bzw. 1 bis 60 Gew.-% des Gesamtgewichts der Zusammensetzung bilden.

10. Verfahren zur kosmetischen Behandlung eines menschlichen oder tierischen Körpers, umfassend, daß man auf den Körper eine Zusammensetzung nach einem der vorhergehenden Ansprüche aufträgt.

## Revendications

1. Une composition cosmétique comprenant au moins trois phases distinctes et séparées, caractérisée en ce que la première phase comprend un organopolysiloxane volatil à faible viscosité, la seconde phase est une phase aqueuse et la troisième phase comprend un fluide de fluorosilicone.

2. Une composition selon la revendication 1 dans lquelle le fluide de fluorosilicone est un polyfluoroalkylméthylsiloxane.

3. Une composition selon la revendication 1 ou 2 dans laquelle le fluide de fluorosilicone est du polyméthyl-3,3,3-trifluoropropyl-siloxane ayant une viscosité comprise entre 300 et 10.000 m²/s à 25° C.

4. Une composition selon l'une quelconque des revendications précédentes dans laquelle l'organo-polysiloxane volatil à faible viscosité comprend un organopolysiloxane cyclique ayant la formule [(CH₃)₂SiO]ₓ dans laquelle x est un nombre entier ayant une valeur comprise entre trois et dix.

5. Une composition selon la revendication 4, dans laquelle l'organopolysiloxane volatil à faible viscosité est un mélange d'octaméthylcyclotétrasiloxane et de décaméthylcyclopentasiloxane.

6. Une composition selon l'une quelconque des revendications précédentes dans laquelle l'organopoly-siloxane volatil à faible viscosité comprend un organopolysiloxane linéaire ayant la formule R²₃SiO(R²₂SiO)ₙSiR²₃ dans laquelle R² est un radical alkyle ayant de un à six atomes de carbone et n est un nombre entier ayant une valeur comprise entre deux et neuf.

7. Une composition selon l'une quelconque des revendications précédentes dans laquelle la première phase comprend un polysiloxane phénylfonctionnel.

8. Une composition selon l'une quelconque des revendications précédentes dans laquelle la première et/ou la troisième phases comprennent aussi des matières hydrophobes et dans laquelle la seconde phase comprend aussi des matières hydrophiles.

9. Une composition selon l'une quelconque des revendications précédentes dans laquelle la première, la seconde et la troisième phases constituent respectivement 1 à 50 pour cent, 1 à 40 pour cent et 1 à 60 pour cent en poids du poids total de la composition.

10. Un procédé de traitement cosmétique des corps humains ou animaux comprenant l'application sur les corps d'une composition selon l'une quelconque des revendications précédentes.
